# EUROPEAN PATENT APPLICATION

(11) **EP 2 491 957 A1**
(43) Date of publication of application: **29.08.2012**
(21) Application number: 10806422.1
(22) Date of filing: 02.08.2010
(51) Int. Cl.: A61L 27/00, C12N 5/077

(54) **ISLET CELL SHEET, PROCESS FOR PRODUCTION THEREOF, AND USE THEREOF**

(30) Priority: 02.08.2009 JP 2009193648
(71) Applicant: Tokyo Women's Medical University, Tokyo 162-8666 (JP)
(72) Inventor: SHIMIZU, Hirofumi, Fukushima-shi Fukushima 960-1295 (JP); OHASHI, Kazuo, Tokyo 162-8666 (JP); UTOH, Rie, Tokyo 162-8666 (JP); ISE, Kazuya, Fukushima-shi Fukushima 960-1295 (JP); YAMATO, Masayuki, Tokyo 162-8666 (JP); OKANO, Teruo, Tokyo 162-8666 (JP); GOTOH, Mitsukazu, Fukushima-shi Fukushima 960-1295 (JP)
(74) Representative: HOFFMANN EITLE
(86) International application number: PCT/JP2010/063033
(87) International publication number: WO 2011/016423

(57) **Abstract**

A polymer that changes hydration at a temperature between 0 to 80°C is coated on the surface of a cell culture support, and islet cells are cultured on the support at a temperature range that causes polymer to have weak hydration, then the temperature of a culture solution is changed to a temperature that causes the polymer to have strong hydration to obtain islet cells in a sheet form. Such islet cells in a sheet form have an insulin producing function even if there is no blood flow.

## Description

### TECHNICAL FIELD

The present invention relates to an islet cell sheet useful in fields including medicine, biology, drug development and pharmacy, a process for the production thereof and a use thereof.

### BACKGROUND ART

The pancreas is an important organ in a living body, which is composed of an exocrine gland that secretes amylase and other digestive enzymes into the duodenum and of islets. Ninety percent or more of the pancreas is occupied by the exocrine gland. Islets which are masses of endocrine gland float like islands in the exocrine gland. An islet is composed of four types of endocrine gland cells that secrete substances in the pancreas, which is an animal organ. The four types of cells are an α cell (A cell) secreting glucagon, a β cell (B cell) secreting insulin, which is a hormone that decreases the blood glucose level, a δ cell secreting somatostatin and a PP cell secreting pancreatic polypeptide. The β cell is a spherical endocrine gland tissue dispersed in the pancreas of various vertebrate animals. It mainly secretes insulin and adjusts the blood glucose level. The diameter of an islet is 100 to 300 µm. A human is said to possess 10 to 20 islets in 1 mg of pancreas and a million or more islets in the whole pancreas. A rodent's islet has β cells situated in the center, and α cells, δ cells and PP cells situated in the periphery, but a human's islet does not have such clear cell distribution.

A damaged islet function leads to severe diseases. An example is the complete loss of insulin secretion by β cells which leads to severe diabetes. Such severe diabetes cases are often accompanied by extreme difficulty in blood glucose control, and also by diabetes-related complications as well as hypoglycemic attack following insulin administration; accordingly, they lead to extremely low quality of life (QOL).

Various treatments have been applied to patients of such severe diabetes. One such treatment, namely, an islet transplantation is recently receiving attention, because the islet transplantation is a treatment method for stably supplying insulin and because it is minimally invasive compared to transplanting the actual organ, namely, the pancreas. Moreover, the introduction of tissue engineering technique has led to present developments including, for example, a method for producing islets in an *in vitro* honeycomb-like porous material of Patent Document 1, and a method for producing islets in a three-dimensional culture device of Patent Document 2. Techniques related to islet transplantation are studied by many researchers, but the fact that the cells used in the transplantation are islets or islet-like masses presently necessitates the cells to be transplanted to regions having abundant blood flow, such as the interior of the portal vein and other blood vessels or hepatic tissues, to supply nutrients and oxygen to tissues inside the masses. Despite the recent establishment of protocols concerning transplantation methods and the consequential improvement of treatment results, the insulin withdrawal of diabetes patients after one year is still only about 45% (refer to Non-Patent Document 1). The problems of this technique include instant blood-mediated inflammatory reaction (IBMIR) and the inflammatory reaction associated with ischemia at the embolic portal vein area due to islets. These problems would result in a loss of over half of the transplanted islets (refer to Non-Patent Documents 2, 3, and 4). Further tissue engineering processing on islets is desired to solve this problem.

Based on the above background, Patent Document 3 teaches a novel method for cell culture that enables cultured cells to be detached without enzyme processing. Such detachment is enabled by culturing the cells on a support covered with a polymer of a temperature no higher than the upper critical solution temperature or a temperature no lower than the lower critical solution temperature, wherein the upper or lower critical solution temperature of the polymer to water is 0 to 80°C, then respectively changing the polymer temperature to the upper critical solution temperature, or higher or to the lower critical solution or lower. Further, Patent Document 4 teaches enabling cultured dermal cells to be detached with little damage by using a temperature responsive substrate for cell culture to culture the dermal cells at a temperature no higher than the upper critical solution temperature or a temperature no lower than a lower critical solution temperature, then changing the substrate temperature to the upper critical solution temperature or higher or the lower critical solution temperature or lower. The use of temperature responsive substrate for cell culture has brought about various new developments from the conventional culture technology. Patent Document 5 further advanced the technology and demonstrated that maintaining the function of the liver tissue cells for long periods, which was difficult in the conventional art, is possible by using the parenchymal cells in the liver tissue as the cell sheet. However, no study has been conducted for islets which have a special morphology.

### PRIOR ART DOCUMENTS

### PATENT DOCUMENTS

[Patent Document 1] Japanese Patent Open Publication (Koukai) No. 2008-278769
[Patent Document 2] Japanese Patent Open Publication (Koukai) No. 2006-304791
[Patent Document 3] Japanese Patent Open Publication (Koukai) No. H02-211865
[Patent Document 4] Japanese Patent Open Publication (Koukai) No. H05-192138
[Patent Document 5] WO 2007/080990

### NON-PATENT DOCUMENTS

[Non-Patent Document 1] The New England Journal of Medicine, 355, 1318-1330 (2006)
[Non-Patent Document 2] Xenotransplantation, 14(4), 288-297 (2007)
[Non-Patent Document 3] Journal of Leukocyte Biology, 77(5), 587-597 (2005)
[Non-Patent Document 4] Pharmacological Reviews, 58, 194-243 (2006)

### SUMMARY OF THE INVENTION

### PROBLEM TO BE SOLVED BY THE INVENTION

The present invention was made to solve the problems relating to the islet transplant technology mentioned above. That is, the present invention provides a novel islet cell sheet based on a completely different idea than the conventional art, the production method thereof and the method of use thereof.

### MEANS TO SOLVE THE PROBLEM

The present inventors performed research and development to solve the above problem by studying it from various angles. They consequently found that destroying the configuration of an islet and reconstructing it into a sheet would free it from the need to be transplanted to the interior of the blood vessel and enable its engraftment to tissues having poor blood flow. The present invention was achieved based on such findings.

In other words, the present invention provides an islet cell sheet that is transplantable to regions other than the interior of the blood vessel and that has an insulin production function. Further, the present invention provides a method for creating the islet cell sheet on a substrate surface covered with a temperature responsive polymer. The present invention further provides a method for using the obtained islet cell sheet. The present invention is a significantly important invention that can be achieved only by the use of a cell structure called a cell sheet, which is based on a unique, novel idea.

### ADVANTAGEOUS EFFECT OF THE INVENTION

An islet cell sheet shown in the present invention frees islet cells from the need to be transplanted in the blood flow, such as in the blood vessel, and enables islet cells to fully exhibit their function, even if the islet cells are transplanted to tissues with poor blood flow, such as the subcutaneous tissue.

### BRIEF DESCRIPTION OF THE DRAWINGS

[Figure 1] Figure 1 is a schematic diagram of Example 1.
[Figure 2] Figure 2 is a schematic diagram of cell sheet detachment in Example 1.
[Figure 3] Figure 3 shows an islet cell cultured on a temperature responsive culture plate in Example 1, at a magnification of 100x.
[Figure 4] Figure 4 is a diagram of stained insulin present in an islet cell that has been cultured on a temperature responsive culture plate in Example 1, at a magnification of 400x.
[Figure 5] Figure 5 shows the result of analyzing insulin secretion according to the sugar concentration of the culture medium in Example 1.
[Figure 6] Figure 6 shows detaching an islet cell sheet cultured on a temperature responsive culture plate in Example 1.
[Figure 7] Figure 7 shows an HE stained islet cell sheet obtained in Example 1, at a magnification of 400x.
[Figure 8] Figure 8 shows the result of staining insulin in the islet cell sheet obtained in Example 1, at a magnification of 400x.
[Figure 9] Figure 9 shows the transplantation of an islet cell sheet obtained in Example 1 to a subcutaneous space on a dorsal site of a rat.
[Figure 10] Figure 10 shows the result of staining insulin in the islet cell sheet extracted from the transplantation site in Example 1.
[Figure 11] Figure 11 shows the change of the glucose amount in blood (concentration) after an islet cell sheet has been transplanted to a diabetic SCID mouse in Example 4 (Title: Change in Blood Concentration of Glucose after Rat Islet Cell Sheet Transplantation in Streptozotocin-Induced Diabetic SCID Mice).

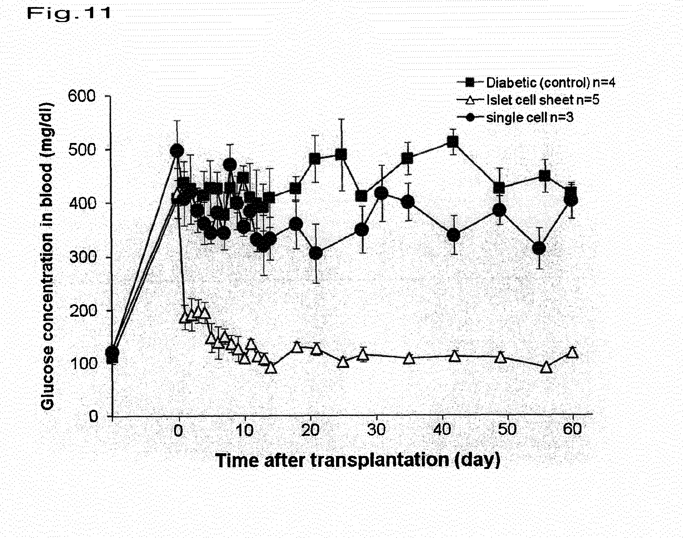

Note that the graph of Figure 11 shows data according to the following condition. Non fasting blood glucose levels of the streptozotocin-induced diabetic SCID mice after transplantation. At day 0, 2, islet cell sheets (triangle: n=5) or single islet cells (circular: n=3) were transplanted into the subcutaneous space on the dorsal site of diabetic SCID mice. Sham-operation (square: n=4). Data are showed as a mean ± SEM. *P < 0.05 (Student's *t* test)
That is, Figure 11 shows the change in the amount of glucose in blood of streptozotocin-induced diabetic SCID mice, which are not fasting, after transplantation of the rat islet shell sheet. An islet cell sheet (triangle: n=5) or an individual islet cell (black circle: n=3) was transplanted to the subcutaneous space on a dorsal site of a diabetic SCID mouse on day zero and after two days. The control was a mouse that underwent sham operation (square: n=4). The data were shown as a mean ± SEM. P<0.05 (t test of Chewdent).

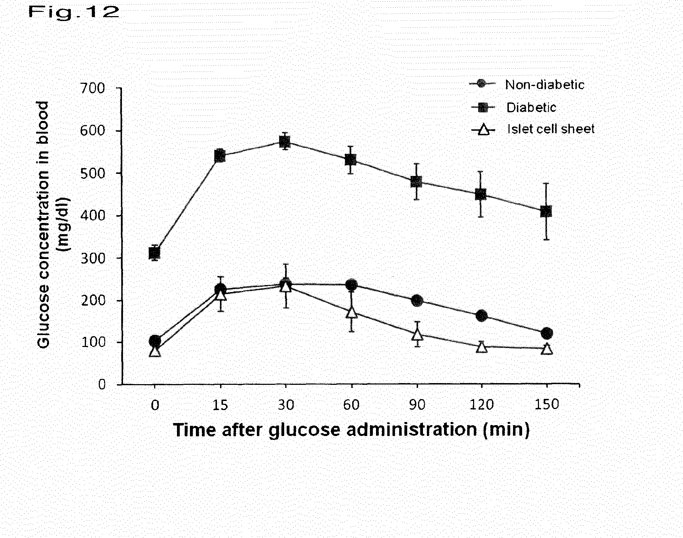

[Figure 12] Figure 12 shows the change in the amount of glucose in blood (concentration) during the glucose tolerance test after the islet cell sheet had been transplanted to a diabetic SICD mouse in Example 5 (Title: Glucose Tolerance Test).

Note that the graph of Figure 12 shows data according to the following condition. Intraperitoneal glucose tolerance test (IPGTT, 2g of glucose/kg body wt) of the streptozotocin-induced diabetic or non-diabetic SCID mice that received 2 islet cell sheets. IPGTT was performed 60 days after islet cell sheet transplantation. IPGTT results showing glucose concentrations before (time 0) and 15, 30, 60, 90, 120, 150 min after glucose administration. Diabetic SCID mice with 2 islet cell sheets (n=7, triangle), diabetic SCID mice with sham-operation (n=5, square), non-diabetic SCID mice without islet cell sheet transplantation (n=11, circle). Data were represented as a mean ± SEM.
That is, Figure 12 shows the result of intraperitoneal glucose tolerance test (IPGTT, 2 g of glucose for 1 kg of body weight) of streptozotocin-induced diabetic or non-diabetic SCID mice that had 2 islet cell sheets transplanted to them. The IPGTT was conducted 60 days from the islet cell sheet transplantation. The IPGTT result shows the glucose concentration before glucose was administered (0 min.) and those 15 min., 30 min., 60 min., 90 min., 120 min., and 150 min. after glucose was administered. The mice used in the test were a diabetic SCID mouse with two islet cell sheets (n=7, triangle), a diabetic SCID mouse with sham operation (n=5, square), and a non-diabetic SCID mouse (n=11, black circle) that had an islet cell sheet transplant. The data were shown as a mean ± SEM.

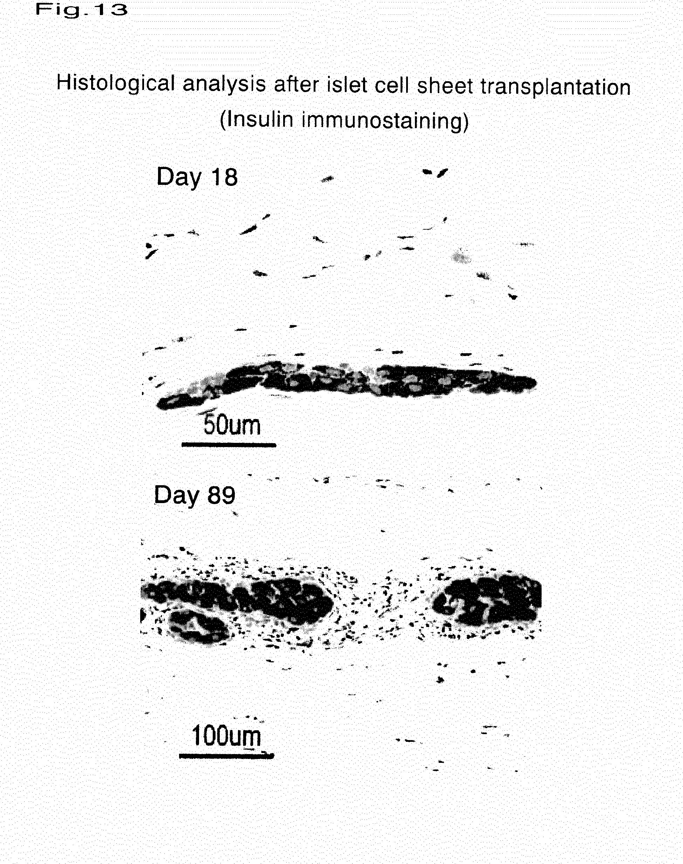

[Figure 13] Figure 13 shows the islet cell sheet after the islet cell sheet was transplanted to a diabetic SCID mouse in Example 6 (Title: Histological Analysis after Islet Cell Sheet Transplantation (Insulin Immunostaining)).

### BEST MODE FOR CARRYING OUT THE INVENTION

The islet cells used in the islet cell sheet of the present invention were collected from the pancreas. The present invention only requires that β cells exhibiting an insulin-producing function are included in those cells, and the content ratio of β cells is not limited. However, considering that the islet cell sheet of the present invention was originally intended for use in producing insulin, the content ratio of β cells should be preferably 40% or higher, more preferably 50% or higher and even more preferably 60% or higher. No limitation concerning types and ratios exists for including a cell which is not a β cell, and cells included in islets, namely, an α cell, a δ cell and a PP cell, can also be included. If the islet cell sheet of the present invention includes an α cell, the sheet will also produce glucagon, and if the islet cell sheet includes a PP cell, the sheet will secrete pancreatic polypeptide; such sheets are preferable because they exhibit functions that are more islet-like. The islet cell sheet of the present invention will detect the sugar concentration of the area surrounding the islet cell sheet, and according to the sugar concentration, produce insulin and/or other physiologically active substances that the islet produces. Cells which are not included in an islet can be included in the present invention without any limitation to the type. Such cells can be a single type or a mixture of two or more types of cells selected from a Sertoli cell, a pancreatic duct cell, a vascular endothelial cell, an endothelial progenitor cell, a hepatic parenchymal cell, a bone marrow derived cell, and a fat derived cell. The content ratio of each of the above cells is not particularly limited either.

The cells used in the present invention include without any limitation cells directly collected from a tissue of a living body, cells or cell strains collected from the organic tissue, then differentiated in a culture system, such as induced pluripotent stem cells (iPS cells), embryonic stem cells (ES cells). The cells are derived from, for example, human or rat, mouse, guinea pig, marmoset, rabbit, dog, cat, sheep, pig, chimpanzee, or immunodeficient animals thereof. Islet cells of the present invention to be used for treating a human should preferably be cells derived from human, pig, and chimpanzee. The media for cell culture of the present invention are not particularly limited as long as those media are commonly used for the cells to be cultured, such as RPMI 1640.

The present invention requires that the cells in the islet are separated by enzyme treatment. The islet is a stable cell mass, but the fact that the cells used in the transplantation are cell masses necessitated the cells to be transplanted to regions having abundant blood flow, such as the interior of the blood vessel. The present invention further separates such stable islet to individual cells, and such separating operation may cause small damage to useful cells such as the β cell. However, the obtained islet cell sheet is not thick like the cell mass, and the sheet does not need to be transplanted to the interior of the blood vessel, which has abundant blood flow, as the cell mass does. The treatment only needs to follow common procedures; there are no other limitations. The number of cells to be disseminated while culturing varies by the animal type, but it should be generally 0.4×10⁶ to 2.5×10⁶ units/cm², preferably 0.5×10⁶ to 2.1×10⁶ units/cm², and more preferably 0.6×10⁶ to 1.7×10⁶ units/cm². A dissemination concentration that is lower than 0.4×10⁶ units/cm² leads to poor growth of islet cells that suppresses the development level of the function of the obtained islet cell sheet, so such concentration is not preferable in working the present invention. Further, a dissemination concentration that is higher than 2.5×10⁶ units/cm² also leads to poor growth of islet cells that suppresses the development level of the function of the obtained islet cell sheet, so such concentration is not preferable in working the present invention.

In the present invention, a polymer that changes hydration at a temperature between 0 to 80°C is coated on the surface of a cell culture support, and the above cells are cultured on the support at a temperature range that causes the polymer to have weak hydration. The preferable temperature is 37°C, which is a common temperature for culturing cells. The temperature reactive polymer used in the present invention can be a homopolymer or a copolymer. Such polymer includes a polymer of Japanese Patent Open Publication (Koukai) No. H02-211865. Such polymer can be specifically obtained by, for example, homopolymerization and copolymerization of the following monomers. Monomers for use include (meth)acrylamide compound, N-(or N,N-di)alkylsubstituted (meth)acrylamide derivative, or a vinyl ether derivative, and any two of these can be used to form a copolymer. Further, a copolymerization of the above monomers with other monomers, or a graft or copolymerization of polymers among themselves, or a mixture of a polymer and a copolymer can be used. Furthermore, polymers can be cross-linked to the extent that the intrinsic property of the polymer is not lost. The above process uses cells, separated at 5°C to 50°C, as the object to be cultured and detached. Temperature responsive polymers that can be used in such process include poly-N-n-propylacrylamide (21°C as lower critical solution temperature of a homopolymer), poly-N-n-propylmethacrylamide (27°C as lower critical solution temperature of a homopolymer), poly-N-isopropylacrylamide (32°C as lower critical solution temperature of a homopolymer), poly-N-isopropylmethacrylamide (43°C as lower critical solution temperature of a homopolymer), poly-N-cyclopropylacrylamide (45°C as lower critical solution temperature of a homopolymer), poly-N-ethoxyethylacrylamide (about 35°C as lower critical solution temperature of a homopolymer), poly-N-ethoxyethylmethacrylamide (t about 45°C as lower critical solution temperature of a homopolymer), poly-N-tetrahydrofurfurylacrylamide (about 28°C as lower critical solution temperature of a homopolymer), poly-N-tetrahydrofurfurylmethacrylamide (about 35°C as lower critical solution temperature of a homopolymer), poly-N,N-ethylmethylacrylamide (56°C as lower critical solution temperature of a homopolymer), poly-N,N-diethylacrylamide (32°C as lower critical solution temperature of a homopolymer). Monomers used for copolymerization in the present invention include without limitation water-containing polymers of polyacrylamide, poly-N,N-diethylacrylamide, poly-N,N-dimethylacrylamide, polyethylene oxide, polyacrylic acid and its salt, polyhydroxyethyl methacrylate, polyhydroxyethyl acrylate, polyvinyl alcohol, polyvinylpyrrolidone, cellulose, carboxymethyl cellulose.

The method for coating the substrate surface with the above polymers is not particularly limited, but the substrate can be coated by subjecting the substrate and the above monomer or polymer to one of electron beam irradiation (EB), γ ray irradiation, UV ray irradiation, plasma treatment, corona treatment, and organic polymerization, or by physical adsorption including coating and kneading, or by other methods. The amount of temperature responsive polymer coated on the surface of the culture substrate should be 1.1 to 2.3 µg/cm², preferably 1.4 to 1.9 µg/cm², and more preferably 1.5 to 1.8 µg/cm². A coated amount lower than 1.1 µg/cm² makes it difficult to detach the cells from the polymer even with stimulation; such amount significantly suppresses the operational efficiency and is not preferable. On the contrary, a coated amount of 2.3 µg/cm² or higher makes it difficult for cells to attach to the region; hence, cells cannot be sufficiently attached. However, the amount of temperature responsive polymer to be coated onto the substrate surface can be 2.3 µg/cm² or higher if the top of the temperature responsive polymer coating layer is further coated with cell-adhesive proteins; then, the amount of temperature responsive polymer to be coated should be 9.0 µg/cm² or lower, preferably 8.0 µg/cm² or lower, and advantageously 7.0 µg/cm² or lower. A coated amount of temperature responsive polymer that is 9.0 µg/cm² or higher makes it difficult for cells to attach to the surface, even if cell-adhesive proteins are further coated on top of the temperature responsive polymer coating layer; hence, such amount is not preferable. The types of cell-adhesive proteins to be used are not limited, but they include collagen, laminin, laminin 5, fibronectin, Matrigel™ used alone or as a mixture of two or more thereof. The method for coating such cell-adhesive proteins is acceptable as long as it follows the conventional method, and normally, the substrate surface is coated with an aqueous solution of the cell-adhesive protein, then the solution is removed and the substrate is rinsed. The present invention is a technique attempting to use the cell sheet per se by using a temperature responsive culture plate. Hence, it is not preferable for the amount of cell-adhesive proteins coated on the temperature responsive polymer layer to be excessively large. The amount of temperature responsive polymer and the amount of cell-adhesive proteins can be measured according to conventional methods. Such methods include directly measuring the cell adhering section using FT-IR-ATR and a method for determining the amounts based on the amount of labeled polymer fixed on the cell-adhering section, wherein the labeled polymer is fixed using a method similar to the above, and either method can be employed.

In order to detach and collect the cultured cell sheet from the temperature responsive substrate in the method of the present invention, the cell sheet can be detached by raising the temperature of the culture substrate to the higher critical solution temperature of the polymer coated on the culture substrate or higher, or lowering the same temperature to the lower critical solution temperature thereof or lower, wherein the culture substrate is the one that the cultured cells adhered to. This process can be performed in the culture medium or other isotonic solutions, wherein the solutions can be selected according to the purpose. Other methods, such as lightly tapping or swaying the substrate, or further, stirring the culture medium using a pipet, can be used alone or in combination to detach and collect the cells more quickly and efficiently. Culture conditions other than the temperature are not particularly limited as long as conventional methods are taken. Examples of culture media to be used include a culture having a fetal calf serum (FCS) and other serums known in the art added to them, or it may be a serum-free culture medium that has no such serum added to it.

The above matter is described using poly(N-isopropylacrylamide) as an example of a temperature responsive polymer. Poly(N-isopropylacrylamide) is known to be a polymer having a lower critical solution temperature of 31°C. A free poly(N-isopropylacrylamide) is dehydrated in water at 31°C or higher, then its polymer chains aggregate and the water becomes white. On the contrary, the polymer chains are hydrated at 31°C or lower to dissolve in water. In the present invention, the polymer is coated and fixed on the surface of a substrate, such as a schale. Accordingly, the polymer on the surface of the substrate is similarly dehydrated at a temperature of 31°C or higher, and the substrate surface becomes hydrophobic, because the polymer chains are coated and fixed on the surface of the substrate. On the contrary, the polymer on the substrate surface is hydrated at a temperature of 31°C or lower, and the substrate surface becomes hydrophilic, because the polymer chains are coated and fixed on the surface of the substrate. The hydrophobic surface above is suitable for cell adhesion and proliferation, and the hydrophilic surface becomes a surface that cells cannot adhere to, so cells or cell sheets being cultured can be detached by merely being cooled.

Compounds commonly used in cell culture, such as glass, modified glass, polystyrene, polymethylmethacrylate, and substances that can be generally shaped, such as polymer compounds other than the above, and ceramics can all be used as a substrate to be coated.

The shape of a culture substrate in the present invention is not particularly limited, and the substrate includes that having the shape of a dish, multi-plate, flask, cell insert, or that of a flat membrane shape.

An islet cell sheet that was created on the temperature responsive substrate, and that was not damaged at culture by proteolytic enzymes, typically dispase and trypsin, is used in the present invention. Hence, the islet cell sheet detached from the substrate contains adhesive protein. Accordingly, islet cells detached in a sheet form maintains some desmosome structure between cell-cell. This structure allows adequate adhesion to the affected tissue when the cells are transplanted, and allows efficient transplant to be performed. Generally speaking, dispase, which is a proteolytic enzyme, is known to detach the cells while maintaining 10 to 40% of the desmosome structure between cell-cell, but the basement membrane protein and the like between cell-substrate are mostly destroyed, so only a weak cell sheet can be obtained. On the contrary, the islet cell sheet of the present invention maintains 60% or higher of both the desmosome structure and the basement membrane protein; hence, the above various effects can be obtained.

The islet cell sheet of the present invention is thus obtained. A plurality of isolate shell sheets can be laminated or stratified in the present invention. The number of sheets to be stratified is not particularly limited, but the number of times the sheets are stratified should be ten times or lower, preferably eight times or lower and more preferably four times or lower. Laminating islet cell sheets improves the cell density of a unit sheet area and the function of the islet cell sheet; thus, such lamination is preferable.

The stratified cell sheet used in the present invention can be stratified in combination with sheets consisting of other cells, such as cell sheets of cells that introduce blood vessels into the cell sheets including vascular endothelial cells or endothelial progenitor cells, or cartilage cell sheets for immunoisolation of islet cell sheets. In such combination, the use of two or more types of different cells preferably induces different cells to interact and results in cell sheets with higher activities. Cells to be used in such combination are not particularly limited, and a cell sheet comprising a single type or a mixture of two or more types of cells selected from a Sertoli cell, a cartilage cell, a pancreatic duct cell, a vascular endothelial cell, an endothelial progenitor cell, a hepatic parenchymal cell, a bone marrow-derived cell, and a fat-derived cell can be used. The position, order and number of lamination are not particularly limited, but such conditions can be changed in accordance to the tissue subjected to coating or anaplerosis. Conditions can be changed as necessary, for example, by using synovial membrane-derived cell sheet having strong adhesion. Further, the number of lamination should be no more than ten times, preferably no more than eight times, and more preferably no more than four times. When vascular endothelial cells are selected in such lamination, excess lamination can be prevented by a method of constructing vascular plexus in a stack of cell sheets. The construction methods of the vascular plexus are not particularly limited, but such methods include a method of mixing vascular endothelial cells within a stack of cell sheets in advance, a method of stratifying vascular endothelial cell sheets when producing a stack of cell sheets, or a method of constructing the vascular plexus by embedding a stack of cell sheets in the living body.

The method of creating a laminate of cell sheets of the present invention is not particularly limited, but such a laminate of cell sheets can be obtained by detaching cultured cells in a sheet form and using an implement for transferring cultured cells to stack cultured cell sheets as necessary. The temperature of the culture medium is not particularly limited as long as it is within the following ranges: when the above polymer coated on the culture substrate surface has a higher critical solution temperature, the temperature of the culture medium in the above process should be the higher critical solution temperature or lower; when the above polymer has a lower critical solution temperature, the temperature of the culture medium should be the lower critical solution temperature or higher. However, a low temperature range which prevents proliferation of cultured cells or a high temperature range which annihilates cultured cells is unsuitable for culture. Culture conditions other than temperature are not particularly limited as long as they follow conventional methods. For example, a culture medium having fetal calf serum (FCS) and other serums known in the art added to them may be used, or a serum-free culture medium that has no such serum added to it may be used. The implement for transferring cultured cells to be used is not limited as long as it can capture detached cell sheets; such implements include films, plates or sponges, such as a porous film, paper or rubber. Implements having handles to facilitate stratification activities can be used with films, plates or sponges, such as a porous film, paper or rubber attached to it.

Islet cell sheets can be coated with synthetic polymer or natural polymer, or they can be microencapsulated according to conventional methods in the present invention to stabilize the function of islet cell sheets. The methods of coating or microencapsulating the polymer in the above process and the materials to be used therein are not particularly limited, but materials, such as polyvinyl alcohol, urethane, cellulose and its derivative, chitin, chitosan, collagen, polyvinylidene difluoride (PVDF), silicon, are shaped into a film, porous film, nonwoven fabric, or woven fabric to be used in contact with the islet cell sheet.

As described above, the laminate of cell sheets of the present invention is obtained by detaching cultured cell sheets from the cell cultured substrate coated by a temperature responsive polymer and using an implement for transferring cultured cells as necessary; the cultured cell sheet is not damaged by proteolytic enzymes, typically Dispase, trypsin, at culture; the basement membrane protein between cell-substrate formed at culture is not damaged by the enzyme; and, the desmosome structure between cell-cell is maintained; thus, the laminate of cell sheets has little structural faults and is strong.

The carrier used for firmly attaching islet cell sheets is a structure for maintaining the cells of the present invention, and it can be formed by using a polymer membrane or a structure molded from a polymer membrane, a metal implement or other matters. A polymer is an example of a material to be used as the carrier, and specific materials constituting such polymer include polyvinylidene difluoride (PVDF), polypropylene, polyethylene, cellulose and its derivative, papers, chitin, chitosan, collagen, urethane and gelatin. The shape of the carrier is not particularly limited.

The islet cell sheet obtained in the present invention can be transplanted to a predetermined region of the living body. The transplantation site can be anywhere in the living body and there is no particular limitation, but examples of such region include the subcutaneous tissue, the greater omentum, the intraperitoneal tissue, the subperitoneal tissue, the liver, muscles, the subfascial tissue. Of the above regions, the greater omentum is particularly preferable because it has abundant blood vessels and it is an easy region to transplant to. The transplantation sites may have or not have blood vessels induced therein in advance; such matter is not particularly limited. Nor is the method of inducing a blood vessel particularly limited, but examples include a method of embedding FGF, which is a vascular proliferation factor, in a microsphere and making the microsphere act on the living body for 8 to 10 days while changing the composition, size and injection range of the microsphere, and a method of creating a space containing induced blood vessels by cutting the polyethylene terephthalate mesh to a given size, creating a bag, putting FGF that has been dissolved into a high concentration agarose solution, then removing the bag 8 to 10 days later. Either way, the islet cell sheet obtained in the present invention does not need to be transplanted to an area of blood flow, such as the interior of the blood vessel, as in conventional islet transplantations. A conventional islet needs to be transplanted to a region having abundant blood flow, such as the interior of the portal vein and other blood vessels or hepatic tissues, to supply nutrients and oxygen to cells inside the masses. In contrast, the islet cell sheet of the present invention are structured with the islet cells arranged in one layer, so nutrients and oxygen can be supplied easily to cells constituting the cell sheets. Hence, the islet cell sheet of the present invention no longer need to be transplanted to sections with abundant blood stream, such as the interior of the blood vessel, and they can be transplanted to normal tissues other than the interior of the blood vessel. The islet cell sheet of the present invention can develop pancreas functions essential for maintaining life by only a simple transplantation.

The islet cell sheet transplanted in the present invention retains a basement membrane protein, so it exhibits excellent engraftment. The number of cells to be transplanted should be changed according to the purpose, and the total activity of the islet function can be changed by changing the size and shape of the cell sheet when the cells to be transplanted are in a sheet form.

When the islet cell sheet of the present invention is applied to a human, the transplanted islet cell sheet will develop islet functions in the human living body over a long time to naturally form an artificial pancreas. The magnitude of the function to be developed can be regulated by the either one of or both the size and shape of the detached islet cell sheet. Such artificial pancreas is used in a radical treatment of diseases such as type I diabetes, type II diabetes, chronic pancreatitis, or in a treatment after the total extirpation of the pancreas, or to support the pancreatic function, but there is no particular limitation to its usage.

When the islet cell sheet of the present invention is transplanted to an animal, the animal becomes an islet cell transplanted animal. The magnitude of the function to be developed can be regulated by the size and/or shape of the detached islet cell sheet. Animals to be used include without limitation a rat, a mouse, a guinea pig, a marmoset, a rabbit, a dog, a pig, a chimpanzee or immunodeficient animals thereof. These islet transplanted animals are used without limitation specifically for pancreatic function assessment systems that assess the effect of a specimen to the pancreatic function by the administration of the specimen to an islet transplanted animal.

### EXAMPLES

The present invention is described in detail below based on the Examples without being limited thereby.

### Example 1

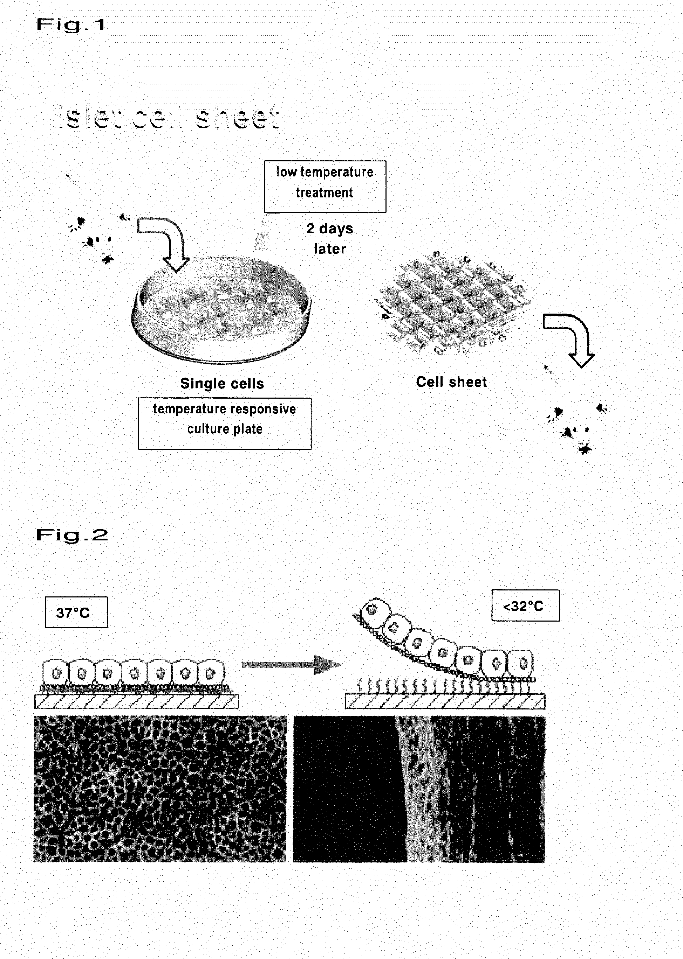

Cells can be recovered in a sheet form by culturing cells from various organs on the temperature responsive culture plate and subjecting them to low temperature treatment after they become confluent. This technology has been applied to cardiac muscles, corneal epithelium, esophageal mucous membrane epithelium, and pancreatic cells, but no study has been conducted on islets so far (Fig. 1).

### (Separation of the Islet Cell)

Laparotomy was performed on an 8 weeks old rat (Lewins rat, male, body weight about 250 g) under isoflurane anesthesia to extract islets. The extracted islets were reacted in a collagenase solution for 32 minutes, then the islets were separated by the concentration gradient method using a Ficoll solution. The obtained islet was reacted for 5 minutes with 0.125% trypsin-EDTA to separate the islets to individual cells. The cells were stored in the RPMI 1640 culture medium (produced by Sigma-Aldrich Co.) comprising 10% fetal calf serum. The activity (viability) of the obtained islet cells was 90% or higher.

### (Culture of Islet Cells)

A 35 mm cell culture plate (produced by Corning Co.) made of polystyrene and coated with poly-N-isopropylacrylamide, which is a temperature reactive polymer, was used as a cell culture substrate. The temperature reactive polymer was coated on to the culture plate according to the method shown in Japanese Patent Open Publication No. H02-211865, and a temperature responsive cell culture substrate having 4.9 µg/cm² of temperature responsive polymer coated thereon was finally obtained. In this experiment, Laminin-5 (Chemicon Co.) was further coated on to the surface of the temperature responsive cell culture substrate in an amount of 0.2 µg/cm². The above mentioned islet cells were disseminated in an amount of 5.7×10⁵ units/cm² on the surface of the coated substrate, then the islet cells were cultured for two days. RPMI 1640 culture medium (produced by Sigma-Aldrich Co.) containing 10% fetal calf serum was used as the culture medium in the above process. The substrate surface having poly-N-isopropylacrylamide coated thereon is hydrophobic at 32°C or higher and hydrophilic at 32°C or lower. Accordingly, cells can adhere to an object during culture at a culture condition of 37°C, and at 37°C or lower, the islet cells are detached in a sheet form without trypsin processing (Figure 2).

### (Assessment of the Islet Cell Sheet)

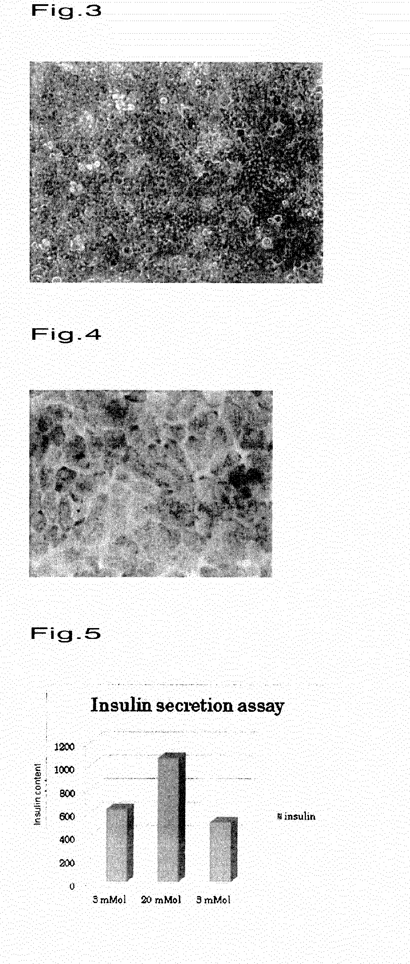

The islet cells on the culture plate reached about 100% in density on the second day of culture (Figure 3). When the islet cells that reached cofluency on this culture plate were observed upon insulin staining, 70 to 80% of the cells were insulin positive (Figure 4). Then, insulin concentrations of islet cells in the culture media were measured for islet cells on temperature responsive culture substrata prepared apart from the above, and the culture media created to have glucose concentrations of 3 mM, 20 mM and 3 mM were used with each culture medium replaced every hour. Insulin secretion ability according to the sugar concentration was proven accordingly (Figure 5). Further, the islet cell sheet was fluorescent stained using PKH26 (SIGMA). Consequently, the obtained cells were found to be islet cells.

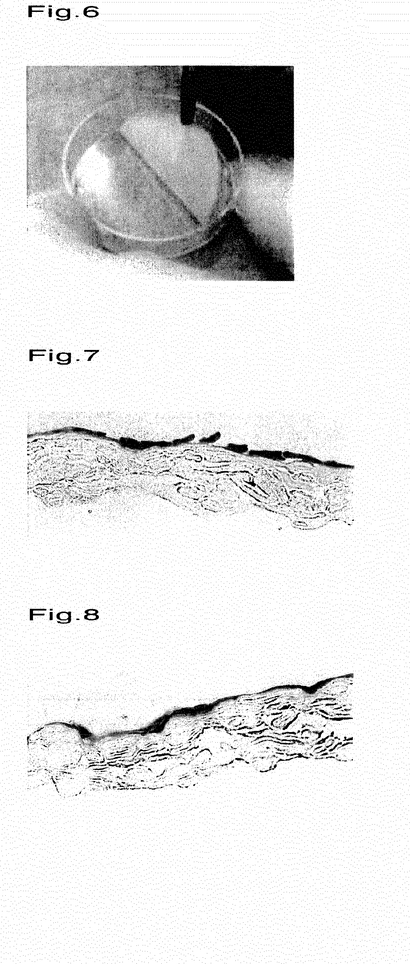

Then, the obtained islet cell sheet was kept static for 30 minutes in a culture room at 20°C and subsequently detached from the surface of the temperature responsive culture substrate by using a support membrane (CellShifter™, produced by CellSeed Co.). No cells remained on the cell plate after the cell sheet was collected (Figure 6). The islet cell sheet attached to the support membrane was embedded in a compound to create a frozen section. The section was observed by HE staining, insulin staining, and using a fluorescent microscope, and the intracellular structure was further observed using an electron microscope. Consequently, the islet cell sheet was observed to be in a form of a single-layer sheet (Figure 7). The islet cell sheet was further observed as being formed from insulin positive cells and insulin negative cells (Figure 8). In addition, the observation by electron microscope showed that the obtained islet cell sheet has no damage in the desmosomal structure and the basement membrane protein.

### (Transplant of Islet Cell Sheets and the Assessment of the Transplant)

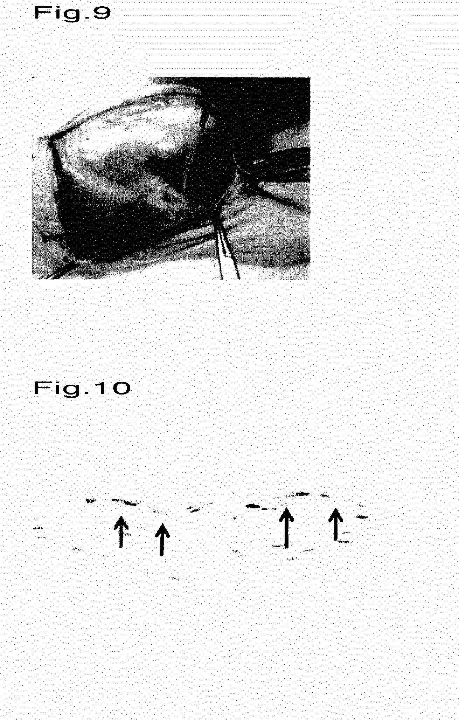

Lastly, the obtained islet cell sheet was transplanted to a rat of the same type as the donor (Lewis rat, 8 weeks old, male, body weight about 250 g). The dorsal skin was incised in an L shape to detach the subcutaneous tissue, and the fascia of that region was exposed. To that section, the above islet cell sheet which was collected using the support membrane was statically placed; after the cell sheet and the surface of the fascia adhered, a normal saline solution was added to the support membrane to slowly remove the support membrane. Adding water to the support membrane lowered the adsorptive power between the support membrane and the islet cell sheet, so the islet cell sheet remained on the fascia, and allowed the sheet to be transplanted without being raffled or moved (Figure 9). The transplanted tissue in the dorsal region was collected 4 days after the transplant, after the mouse was bled to death. The collected tissue in the form of a frozen section was assessed by insulin staining and by using a fluorescent microscope. Consequently, the presence of islet cells in the form of an islet cell sheet was confirmed in the tissue extracted 4 days after transplant by fluorescent staining the islet cells in advance. Further, the presence of insulin positive cells in the cells forming the sheet was confirmed by insulin staining (Figure 10).

### Example 2

An experiment was conducted that was identical to Example 1 other than that the islet cell sheet obtained in Example 1 was transplanted to the greater omentum of a rat of a same type as the donor (Lewis rat, 8 weeks old, male, body weight about 250 g). The tissue of transplant in the greater omentum was collected 14 days after the transplant, after the mouse was bled to death. The collected tissue in the form of a frozen section was assessed by insulin staining and by using a fluorescent microscope. Consequently, the presence of islet cells in the form of an islet cell sheet was confirmed in the tissue extracted 14 days after transplant by fluorescent staining the islet cells in advance. Further, the presence of insulin positive cells in the cells forming the sheet was confirmed by insulin staining.

### Example 3

A vascular endothelial cell sheet created separately on the same temperature responsive culture substrate as Example 1 was stratified on the islet cell sheet obtained in Example 1 to create a stratified cell sheet which stacks in sequence, an islet cell sheet, a vascular endothelial cell sheet and an islet cell sheet. An experiment was conducted that was identical to Example 1 other than that the stratified islet cell sheet was transplanted to the subcutaneous tissue of a rat of the same type as the donor (Lewis rat, 8 weeks old, male, body weight about 240 g). The subcutaneous tissue of transplant was collected 14 days after the transplant, after the mouse was bled to death. The collected tissue in the form of a frozen section was assessed by insulin staining and by using a fluorescent microscope. Consequently, the presence of an islet cell in the form of an islet cell sheet was confirmed in the tissue extracted 14 days after transplant by a prior fluorescent staining of the islet cells. Further, the presence of insulin positive cells in the cells forming the sheet was confirmed by insulin staining. Further, the entry of a blood vessel to the stratified islet cell sheet showed that a thick islet cell sheet has been engrafted.

### Example 4

A diabetic SCID mouse was prepared according to conventional methods. Such mouse was prepared specifically by weighing the SCID mouse, then administering intraperitoneally an amount of streptozotocin (produced by Sigma) directed to a body weight of 220 mg/kg. After streptozotocin was administered, the whole blood was collected from the caudal vein, and the blood glucose level was measured by using a mini glucose-meter Glucocard (produced by Japan Hoechst Marion Roussel Co.). Mice with a blood glucose level of 350 mg/dL or higher was considered as having diabetes. The islet cell sheet of Example 1 was transplanted subcutaneously in a dorsal region of the diabetic SCID mouse by the same procedure as Example 1. Fig. 11 shows the change in the amount of glucose in blood after the transplantation. The amount of glucose in blood does not decrease in a diabetic SCID mouse, but for a mouse having an islet cell sheet transplanted to it, the amount of glucose in blood decreased immediately after transplantation, and the effect lasted at least 60 days, and further, the amount of glucose in blood showed no tendency of increasing again after 60 days. Meanwhile, the same experiment was performed with separate islet cells that are not formed into a sheet, but the amount of glucose in blood did not decrease by such method, nor did the effect last. Accordingly, the islet cell sheet of the present invention is effective in the treatment of diabetes.

### Example 5

A diabetic SCID mouse and a diabetic SCID mouse that had islet cell sheet transplantation after Example 4, as well as a healthy SCID mouse were each further subjected to a glucose tolerance test. The glucose tolerance was set to 2 g/kg of body weight in the test. The obtained result is shown in Figure 12. The result showed that the amount of glucose in blood does not decrease in a diabetic SCID mouse, but a mouse having an islet cell sheet transplanted to it exhibited similar results to the healthy mouse in that the amount of glucose in blood decreased promptly from 30 minutes after the glucose load, and the effect lasted, and further, the amount of glucose in blood showed no tendency of increasing again after 150 minutes. Accordingly, the islet cell sheet of the present invention is effective in the treatment of diabetes.

### Example 6

The mice of Example 4 was bled to death 18 days and 89 days after the islet cell sheet transplantation, and the tissue in the dorsal section receiving islet cell sheet transplantation was collected. The collected tissue was evaluated as a formalin-fixed paraffin-processed section by insulin staining. As a result, islet cells organized in a sheet form in the subcutaneous tissues that the islet cell sheet was transplanted to, and the presence of insulin positive cells were confirmed (Figure 13). The tissues of 89 days after the transplantation showed insulin positive cells rearranging in the transplantation site and gathering together. This result suggests the possibility that the islet cell sheets transplanted in the present invention will be rearranged in a more preferable state.

### INDUSTRIAL APPLICABILITY

The islet cell sheet created on the temperature responsive culture substrate does not require islet cells to be transplanted in the blood vessel, and functions of the islet cells per se can sufficiently develop by transplantation to tissues with poor blood flow, such as subcutaneous tissues.

## Claims

1. An islet cell sheet that is transplantable to a region other than an interior of a blood vessel and that has an insulin producing function.

2. The islet cell sheet according to claim 1, comprising a β cell at content ratio of 40% or higher.

3. The islet cell sheet according to claim 1 or 2, having a glucagon producing function and a glucose concentration detecting function.

4. The islet cell sheet according to any one of claims 1 to 3, comprising a single type of cell or a mixture of two or more types of cells selected from a Sertoli cell, a pancreatic duct cell, a vascular endothelial cell, an endothelial progenitor cell, a hepatic parenchymal cell, a bone marrow derived cell, and a fat derived cell.

5. The islet cell sheet according to any one of claims 1 to 4, wherein the islet cell sheet is formed by stratifying two or more islet cell sheets.

6. The islet cell sheet according to any one of claims 1 to 5, wherein the islet cell sheet is formed by forming two or more cell sheets which are islet cell sheets comprising a single type of cell or a mixture of two or more types of cells selected from a Sertoli cell, a cartilage cell, a pancreatic duct cell, a vascular endothelial cell, an endothelial progenitor cell, a hepatic parenchymal cell, a bone marrow derived cell, and a fat derived cell.

7. The islet cell sheet according to any one of claims 1 to 6, wherein the islet cell sheet is coated by a synthetic polymer and/or a natural polymer.

8. The islet cell sheet according to any one of claims 1 to 7, wherein the islet cell sheet is microencapsulated.

9. A method for producing an islet cell sheet comprising the steps of:
culturing islet cells on a cell culture support having a surface coated with a polymer at a temperature range that causes the polymer to have weak hydration, wherein the polymer is a polymer changing hydration between 0°C and 80°C;
subsequently detaching in a sheet form the islet cells that have been cultured by changing a temperature of a culture solution to a temperature that causes the polymer to have strong hydration.

10. The method for producing an islet cell sheet according to claim 9, wherein the surface of the cell culture support is coated with laminin-5.

11. A method for producing an islet cell sheet according to either claim 9 or 10, further comprising the step of stratifying a detached islet cell sheet on another islet cell sheet structure, and optionally repeating the stratifying step to stratify islet cell sheets.

12. The method for producing an islet cell sheet according to any one of claims 9 to 11, wherein the islet cell sheet is detached from the cell culture support without being treated with proteolytic enzyme in the detaching step.

13. The method for producing an islet cell sheet according to any one of claims 9 to 12, wherein a carrier is firmly attached on cultured cells at a completion of culturing and the sheet is detached with the carrier.

14. The method for producing an islet cell sheet according to any one of claims 9 to 13, wherein islet cells are collected from a tissue of a living body.

15. The method for producing an islet cell sheet according to any one of claims 9 to 14, wherein a number of cells to be disseminated at culture is 0.4×10⁶ to 2.5×10⁶ units/cm².

16. The method for producing an islet cell sheet according to any one of claims 9 to 15, wherein the polymer changing hydration between 0°C and 80°C is poly(N-isopropylacrylamide).

17. A method for using an islet cell sheet comprising a step of transplanting an obtained islet cell sheet to a predetermined region in a living body.

18. The method for using an islet cell sheet according to claim 17, wherein an transplantation site is a subcutaneous tissue, a greater omentum, an intraperitoneal tissue, a subperitoneal tissue, a liver, muscles, a subfascial tissue in a living body.

19. The method for using an islet cell sheet according to either claim 17 or 18, wherein a transplantation site is a region subjected to blood vessel induction in advance.

20. The method for using an islet cell sheet according to claim 19, wherein the blood vessel induction is performed using an FGF process for a long time.

21. An artificial pancreas using an islet cell sheet according to any one of claims 1 to 8.

22. The artificial pancreas according to claim 21, wherein detached islet cells are formed into a sheet, and a size and/or shape of the sheet can regulate a magnitude of function that is developed.

23. The artificial pancreas of either claim 21 or 22 for a treatment of type I diabetes, type II diabetes, chronic pancreatitis, or a treatment after a total extirpation of a pancreas, or for support of a pancreatic function.

24. The artificial pancreas of any one of claims 21 to 23, having an islet cell function that is enhanced by gene transfer techniques.

25. An islet cell transplant animal having an artificial pancreas according to any one of claims 21 to 24 transplanted thereto.

26. The islet cell transplant animal according to claim 25, wherein the animal is a rat, a mouse, a guinea pig, a marmoset, a rabbit, a dog, a pig, a chimpanzee, or immunodeficient animals thereof.

27. A pancreatic function assessment system that assesses an effect of a specimen to a pancreatic function by administering the specimen to the islet cell transplant animal according to claim 25 or 26.
